Europäisches Patentamt

European Patent Office

Office européen des brevets

(11) Publication number: **0 172 163**
**B1**

# EUROPEAN PATENT SPECIFICATION

(45) Date of publication of patent specification: **29.11.89**

(21) Application number: **84900937.8**

(22) Date of filing: **08.02.84**

(86) International application number:
**PCT/US84/00167**

(87) International publication number:
**WO 85/03449 15.08.85 Gazette 85/18**

(51) Int. Cl.⁴: **A 61 N 1/36,** A 61 B 17/56,
A 61 N 1/18

(54) BONE GROWTH STIMULATOR.

(43) Date of publication of application:
**26.02.86 Bulletin 86/09**

(45) Publication of the grant of the patent:
**29.11.89 Bulletin 89/48**

(84) Designated Contracting States:
**AT BE CH DE FR GB LI LU NL SE**

(56) References cited:
EP-A-0 052 247
FR-A-2 220 231
SU-A- 457 470
SU-A- 854 378
US-A-3 783 880
US-A-4 095 602
US-A-4 147 171
US-A-4 285 346
US-A-4 308 863
US-A-4 365 624

ACTA ORTHOP. SCANDINAV., vol. 43, 1972,
pages 421-437 T.E. JÖRGENSEN: "The effect of
electric current on the healing time of crural
fractures"

"The Alternate Treatment of Fracture
Nonunion", issued September, 1979, Zimmer
USA, Lit. No. B-2360-1;

(73) Proprietor: **Zausmed Inc.**
**69 Woodbine Road**
**New York, New York 10956 (US)**

(72) Inventor: **BEN-DOV, Meir**
**69 Woodline Road**
**New York, NY 10956 (US)**

(74) Representative: **Sommerville, John Henry et al**
**SOMMERVILLE & RUSHTON 11 Holywell Hill**
**St. Albans Hertfordshire, AL1 1EZ (GB)**

Courier Press, Leamington Spa, England.

## Description

### Field of the invention

This invention relates to orthopedic surgery and, more specifically, to external fixation and bone growth stimulation apparatus.

### Background of the invention

It has been known for three decades that bone structures have bioelectric properties. It is known, for example, that bones tend to be electronegative in areas of compression and electropositive in areas of tension, and that areas of active growth and repair tend to be electronegative. Many workers have demonstrated the phenomenon of electric current stimulated osteogenesis at the cathode. Electric currents, both AC and DC, including pulsating DC, in the range of from about 10 to 100 microamperes is known to stimulate bone growth in some but not necessarily all subjects. The literature on this subject is extensive, see, e.g. Spadaro JA: Electrically Stimulated Bone Growth in Animals and Man, A Review of the Literature, Clin. Orthop. 122:325, 1977.

Implantable electric current bone growth stimulator devices have been reported, see, e.g., U.S. Patents Nos. 3,745,995; 3,783,880; 3,890,953; 3,915,151; 3,968,790; 4,011,861; 4,052,754; 4,306,564; 4,313,438; 4,315,503; 4,333,469 and 4,414,979. Prostheses having electrically stimulated bone growth devices have also been proposed; see, e.g., U.S. Patents Nos. 3,964,473; 4,195,367; 4,214,322 and 4,216,548. Non-invasive bone growth stimulators, see, e.g. U.S. Patents Nos. 4,056,097; 4,066,065; 4,153,060; 4,175,565 and 4,244,373, and bone growth stimulators with specific current and voltage patterns, see, e.g., U.S. Patents Nos. 4,105,017; 4,266,532; 4,266,533; and 4,315,503, have been described. Semi-invasive bone growth stimulators have also been disclosed, see, e.g., Zimmer, "The Alternate Treatment of Fracture Nonunion, Electrical Stimulation to Induce Osteogenesis, Zimmer USA, Warsaw, Indiana 46580, September 1979 revision, and U.S. Patents Nos. 3,842,841 and 3,918,440.

U.S. Patent No. 4,026,304 reviews the state of the art and early developments, it discusses the problem of polarization and proposes, as a solution, an implantable source of electric potential to generate a train of electric pulses.

U.S. Patent No. 3,893,462 discloses another method of bone growth stimulation utilizing electrical signals undulating in both the positive and negative directions in an asymmetric manner reactively coupled to the bone.

Reference is also made to a paper by T. E. Jorgensen entitled "The Effect of Electric Current on the Healing Time of Crural Fractures" in Acta Orthop. Scandinav., Vol. 43, 1972, pages 421—437. In this paper there is a brief description of so-called Hoffmann apparatus which comprises a solid mounting for the stimulator and electrical electrodes in the bone proximally and distally to the fracture, which electrodes are insulated from each other.

### Summary of the invention

The present invention comprises an apparatus for both fixing a bone fracture and stimulating the bone growth repair of the fracture, while eliminating or at least mitigating the effects of polarization in electric current induced osteogenesis.

According to the present invention, there is provided a combined external fixation and bone growth stimulating means as defined in claim 1.

### Brief description of the drawings

Figure 1 is a side view of the apparatus of this invention, the frame being shown in simplified form.
Figure 2 is an enlarged, fragmentary view of the apparatus shown in Figure 1, and
Figure 3 is a very schematic view illustrating the principle of application of voltage to the cathodes and pins of Figures 1 and 2.

### Description of the preferred embodiment

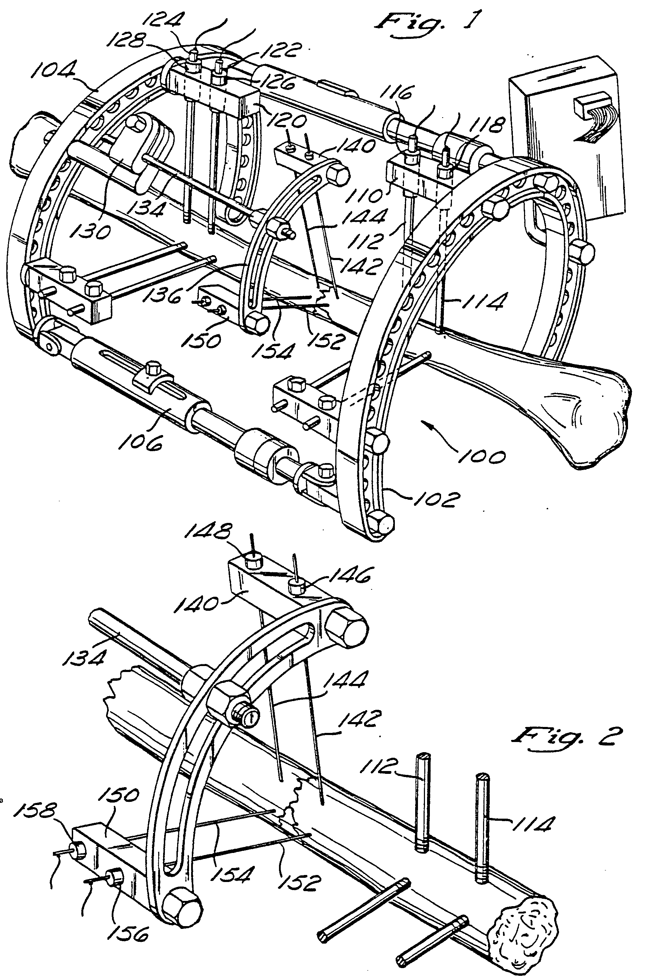

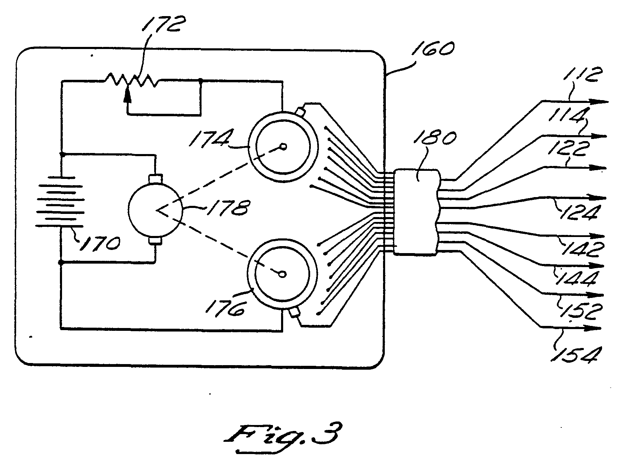

The invention is described as applied to the Ace-Fischer (Trademark) external fixation device, which in very simplified form is shown in Figures 1 and 2; however, it is to be understood and emphasized that the invention includes and comprehends any external fixation device which is capable of fixing fixature pins and cathodes. The Ace-Fischer (Trademark) external fixation device is described in detail in U.S. Patent No. 4,308,863.

The invention includes an external fixation device 100 which may be in any configuration. In the depicted embodiment, which is merely exemplary and non-limiting, the fixation device includes a pair of semicircular frame members 102 and 104 secured in spaced relation about the fractured bone by adjustable rod means one of which is depicted at 106. Pin holders 110 and 120 are secured in any convenient manner to the frame members and fix the fixature pins 112 and 114, in holder 110, and 122 and 124, in holder 120, in position. Electrically insulating means 116 and 118 in holder 110 and means 126 and 128 in holder 120 electrically isolate the pins 112, 114, 122 and 124 from each other such that there is no electrical connection between them through the frame. Insulating means may be, for example, Teflon (Trademark) polytetrafluoroethylene or other insulative sleeves. The distal ends of the pins are screwed, or otherwise secured, in the usual manner to the bone. One pair of pins, 112 and 122, are secured one pin on each side of the fracture site, and the other pair of pins, 114 and 124, are secured also one pin on each side of the fracture site. The pins on each side are spaced apart sufficiently to avoid electrical shorting therebetween.

A bracket 130 secures a rod 134 to the frame means such that the rod extend approximately parallel to the axis of the bone proximate the center of the frame where it supports an arcuate mounting bracket 136. Cathode mounting blocks 140 and 150 are secured to the mounting bracket 136 in a conventional way, such as by a bolt and nut arrangement. The block 140 mounts cathodes 142 and 144 preferable by means of electrically insulative sleeves 146 and 148. In like manner, the block 150 mounts cathodes 152 and 154 by means of sleeves 156 and 158.

As pointed out, the specific structures by which the pins and cathodes are mounted are of no consequence insofar as this invention is concerned so long as they perform the necessary function of mounting the pins in fixed relation with the distal ends of the pins secured to the bone to fix the fracture site of the bone and mounting the cathodes with the distal ends of the cathodes in electrical contact with the bone in the proximity of the fracture site. The tips of the pin may be in the fracture site, in the bone adjacent the fracture site or in the soft tissue adjacent the bone fracture site, all of which locations are referred to herein as being in electrical contact with the bone. The cathodes and pins are electrically isolated from each other, except, of course, through the bone and the source of voltage which will be described, such that a voltage can be applied between any cathode and either pair of pins and between the pairs of pins.

The means for applying a voltage is illustrated for the sole purpose of describing the manner in which the voltage is applied. It will be instantly understood that in practice solid state voltage regulators, switches, etc. will be used. Since the exact circuitry and devices for generating and applying a voltage are of no importance to the operation of the invention, so long as the voltage is applied as described, a simplified schematic representation has been selected to more clearly and simply illustrate the voltage applying means.

As shown in Figure 3, a voltage in a particular cyclical pattern to be described is applied from the voltage applying means 170. Typically, a stable battery having long term constant voltage, indicated at 170, will be used. A current regulator depicted generally at 172 will be included. This, of course, will be a solid state device rather than the functionally schematic variable resister shown. To illustrate the cyclic manner of applying voltage, a pair of wiper switches 174 and 176 driven by motor 178 are shown simply to illustrate that the voltage will be applied sequentially to a number of electrical conductors in cable 180 and thence to the pins 112, 114, 122, and 124, and the cathodes 142, 144, 152 and 154. Again, it is emphasized that solid state switching is conveniently used and that the switching shown is functionally schematic to illustrate the principle. Since solid state circuitry of the type suitable for use in the invention is well known and conventional, and since so many circuits can suitably be used is deemed unnecessary to describe the same in detail. Reference is made to the aforecited patents for various circuits which may used or modified for use. Reference is also made to standard electronic circuitry texts and manuals.

The operation of the voltage apply means is as follows:

In the preferred embodiment, the apparatus includes four cathodes and two sets of pins. The means for applying electrical voltage applies electrical voltage to the cathodes and the pins cyclically for a plurality of time periods during each cycle. The cathodes at all times having either no voltage or negative voltage applied thereto. The pins having either positive, negative or no voltage applied thereto, either the first pins or the second pins being positive when a negative voltage is applied to any cathode. The conductors in cable 180 are connected to the switching mechanism such that the voltage application is cycled to cause electron flow from a first cathode to the first pins in a first period, from the first pins to the second pins during a second period, from the second cathode to the second pins in third period, from the second pins to the first pins in fourth period, from the third cathode to the first pins in the fifth period, from the first pins to the second pins in the sixth period, from the fourth cathode to the second pins in the seventh period, and from the second pins to the first pins in the eighth period.

The connection of the conductors between the switching mechanism and the cathodes and pins and the operation of the switching mechanism is fully defined by the following table.

TABLE I

| Time period | Cathode polarity | | | | Pin polarity | | | |
|---|---|---|---|---|---|---|---|---|
| | 142 | 152 | 144 | 154 | 112 | 114 | 122 | 124 |
| 1 | − | 0 | 0 | 0 | + | + | 0 | 0 |
| 2 | 0 | 0 | 0 | 0 | − | − | + | + |
| 3 | 0 | − | 0 | 0 | 0 | 0 | + | + |
| 4 | 0 | 0 | 0 | 0 | + | + | − | − |
| 5 | 0 | 0 | − | 0 | + | + | 0 | 0 |
| 6 | 0 | 0 | 0 | 0 | − | − | + | + |
| 7 | 0 | 0 | 0 | − | 0 | 0 | + | + |
| 8 | 0 | 0 | 0 | 0 | + | + | − | − |

Current was controlled in the range of 5 to 20 microamperes. The full sequence of pulsing occurs at 10 Hz time intervals. Each sequence involves eight events—four firings (negative charging of a cathode) and four discharges of the anodes (pins). Each of these eight events requires 12.5 milliseconds. Thus, the full eight events requires 100 milliseconds and the sequence repeats itself 10 times each second.

It will, of course, be understood that the specific order of voltage application is not critical and can be altered. What is important is that the electron flow be controlled such that it is always from the cathode to one or the other of the sets of pins, when the cathodes are active, and that there be period flow between the pins opposite the direction of flow when the cathode current flows to the pins. The intensity of the current does not differ from that taught in the prior art and may typically range from about 10 microamps to 100 microamps, normally being from 10 to 20 microamps. These ranges are, of course, typical and not critical.

Discussion

Animal studies of the invention were conducted at the Cleveland Research Institute using a canine model. Torsional strength values were almost double for stimulated tibias as compared with a control series. The histological and microradiographic analysis demonstrated earlier evidence of cellular activity (1—2 weeks post operatively) in the stimulated groups. The 6 weeks post operative analysis showed a more dense and more mature material tibial deposit in the stimulated tibial fractures. Significantly, the incidence of pin loosening was only one-fourth as frequent in the stimulated series as in the control series. Additionally the degree of loosening was 3.5 times greater in the control series as in the stimulated series. The level of trace elements in the model was slightly higher in the stimulated series than in the control, but the difference was marginal and the levels for both groups were well within an acceptable range. It was concluded from this series that the invention was both safe and effective in promoting fracture healing in the canine model. Clinical trials are being planned and it is predicted from the animal tests that the invention will be both safe and effective in promoting human bone growth.

Industrial application

This invention will find industrial application in veterinary medicine and in orthopaedic surgery.

**Claim**

A combined external fixation and bone growth stimulating means comprising a plurality of electrical electrodes positionable in the bone proximally or distally to a fracture and a frame means for mounting the electrodes wherein the growth stimulating means comprises first and second pairs of fixation pins (112, 114, 122 and 124), a plurality of cathodes (142, 144, 152, 154), the frame means (100) for electrically isolating and fixing the position of the pins and cathodes, and which includes means for fixing the first pair of fixation pins (112, 122) one on each side of the fracture site of a bone, the second pair of fixation pins (114, 124) one on each side of the fracture site and the cathodes proximate the fracture site, together with means for applying a voltage for a first period between a cathode and the first pins, during a second period between the first and second pins, during a third period between another cathode and the second pins, and during a fourth period between the second and first pins, the cathode being negative during the first and third periods and neutral during the second and fourth periods, the first pins being negative during the second period and positive during the fourth period.

**Patentanspruch**

Kombinierte Vorrichtung zur äußeren Fixierung und zur Knochenwachstumsanregung, umfassend eine Mehrzahl elektrischer Elektroden, die im Knochen angeordnet sind nahe bei oder entfernt von einer Fraktur, einen Rahmen zum Montieren der Elektroden, wobei die Wachstumsanregungsvorrichtung ein erstes und ein zweites Paar von Befestigungsstiften (112, 114, 122 und 124) umfaßt, eine Mehrzahl von Kathoden (142, 144, 152, 154), den Rahmen (100) zum elektrischen Isolieren und Fixieren der Position der Stifte und der Kathoden, sowie Mittel zum Befestigen des ersten Paares von Befestigungsstiften (112, 122) beidseits der Frakturstelle eines Knochens, des zweiten Paares von Befestigungsstiften (114, 124) beidseits der Frakturstelle, und der Kathoden in der Nähe der Frakturstelle, Mittel zum Anlegen einer Spannung während einer ersten Periode zwischen einer Kathode und den ersten Stiften, während einer zweiten Periode zwischen den ersten und zweiten Stiften, während einer dritten Periode zwischen einer weiteren Kathode und den zweiten Stiften, und während einer vierten Periode zwischen den zweiten und ersten Stiften, wobei die Kathode während der ersten und der dritten Periode negativ, und während der zweiten und vierten Periode neutral ist, und wobei die erste Stifte während der zweiten Periode negativ, und während der vierten Periode positiv sind.

**Revendication**

Dispositif combiné pour la fixation externe et la stimulation de croissance d'un os, comportant une série d'électrodes agencées pour être placées dans l'os à proximité ou à distance d'une fracture et des moyens de structure pour le montage des électrodes, dispositif dans lequel les moyens pour la stimulation

4

**EP 0 172 163 B1**

de croissance comprennent une première et une seconde paire de broches de fixation (112, 114, 122 et 124), une série de cathodes (142, 144, 152, 154), les moyens de structure (100) pour isoler électriquement et fixer en position les broches et cathodes, et lequel comporte des moyens pour fixer la première paire de broches de fixation (112, 122) l'une de chaque côté du site de fracture d'un os, la seconde paire de broches de fixation (114, 124) l'une de chaque côté du site de fracture et les cathodes à proximité du site de fracture, ainsi que des moyens pour appliquer une tension électrique durant une première période entre une cathode et les premières broches, durant une deuxième période entre les premières et les secondes broches, durant une troisième période entre une autre cathode et les secondes broches, et durant une quatrième période entre les secondes et les premières broches, la cathode étant négative durant la première et la troisième période et neutre durant la deuxième et la quatrième période, les premières broches étant négatives durant la deuxième période et positives durant la quatrième période.

5

EP 0 172 163 B1

Fig. 1

Fig. 2

1

Fig. 3